# EUROPEAN PATENT SPECIFICATION

(11) **EP 1 683 540 B1**
(45) Date of publication and mention of the grant of the patent: **24.09.2008**
(21) Application number: 06001187.1
(22) Date of filing: 20.01.2006
(51) Int. Cl.: A61M 25/10, A61M 25/00

(54) **Balloon Catheter having a soft distal tip**
Ballonkatheter mit einer weichen Spitze
Cathéter-ballon pourvu d'une extrémité distale souple

(30) Priority: 21.01.2005 US 646118 P
(43) Date of publication of application: 26.07.2006
(60) Divisional application: 08013262.4
(73) Proprietor: Abbott Laboratories Vascular Enterprises Limited, Terrace, Dublin 2 (IE)
(72) Inventor: Jeffrey, Andrew, 72074 Tuebingen (DE); Balfe, Louise, 70567 Stuttgart (DE); Coffey, Lorcan, 72076 Tuebingen (DE); Unzicker, Kay, 79798 Jestetten (DE); Dzakula, Zdravkica, 8180 Buelach (DE)
(74) Representative: Schmitz, Hans-Werner

(56) References cited:
- EP-A- 1 023 913
- EP-B- 0 365 993
- WO-A-96/09848
- WO-A-03/037419
- US-A- 4 276 874
- US-A- 4 921 483
- US-A1- 2003 032 921
- US-B1- 6 517 515

## Description

### 2. Field of the Invention

The present invention relates to a catheter according to the preamble part of claim 1 for POBA or stent delivery applications. More specifically, the present invention relates to a balloon catheter having a soft distal tip member. Such a catheter is known from EP 1 023 913 A1.

### 3. Background Information

Non-invasive procedures such as percutaneous transluminal angioplasty (PTA), percutaneous transluminal coronary angioplasty (PTCA), stent delivery and deployment, radiation treatment, delivery of a drug at a lesion site and other procedures are used in the treatment of intravascular disease. These therapies are well known in the art and usually utilize a balloon catheter pulled over a guide wire. After a guiding catheter is placed into the patient's main vessel, a guide wire is advanced in the guide catheter and beyond the distal end of the guide catheter. The balloon catheter is then advanced over the guidewire until it reaches the treatment site at the lesion or stenosis. The balloon is inflated to compress the lesion site and dilate the previous narrowed lesion or stenosis site. If the balloon carried a stent and/or drug, the stent and/or drug is delivered at the site when the balloon is inflated. Likewise, further therapies may also use a balloon catheter for the treatment of the lesion site.

Catheters used in vascular procedures must be flexible and soft to navigate safely through tortuous anatomy of the patient's vessels without damaging the vessels, but at the same time they need sufficient stiffness to allow for good pushability and traceability of the catheter. As a result, catheters have been designed to have a more flexible distal end and a stiffer proximal portion. Particularly with regard to the distal part of the catheter, several ways to achieve a soft tip of the catheter were described in US 4782834, US 4921483, US 5964778, US 2002/0188312, US 2003/0114794, US 2003/0032921, and in US 2003/0139761. However, there continues to be a need for a catheter with an extremely flexible and smooth shaped tip that does not diminish trackability of the catheter and allows the balloon portion of the catheter to smoothly cross the lesion. The present invention addresses this need by providing a novel way to attach a flexible distal tip resulting in a new soft tip member and without diminishing from the trackability and pushability of the catheter.

### 4. SUMMARY OF THE INVENTION

It is an object of the present invention to provide a catheter with a highly flexible and soft tip member at the distal end portion of the catheter.

The invention is directed to a balloon catheter according to the features of claim 1 to solve the above object. The dependent claims contain advantageous embodiments of the invention.

The present invention is also directed to a one-step method of forming the tip member generally comprises the steps of positioning the distal balloon shaft over the guide wire lumen, imposing a proximal portion of the soft tip tube on the distal portion of the guide wire lumen tube in a way that the proximal end of the soft tip portion is juxtaposed to the distal end of the balloon cone, and fusion bonding the balloon distal shaft section, the guide wire lumen tube and the soft tip portion to each other. In an alternative embodiment, the proximal portion of the soft tip tube is imposed on the guide wire lumen as well as over a distal end portion of the balloon shaft.

The catheter according to the present invention, having a soft distal tip member attached to the outer surface of the distal portion of the guide wire lumen and being juxtaposed to the distal end of the balloon shaft section or being attached to the outer surface of the distal balloon shaft section as well, shows superior performance with regards to trackability and crossability. With the tip member having a smooth conical shape, the catheter of the present invention has a smooth transition in stiffness and profile from the balloon cone to the distal end of the tip member, thus increasing tensile strength, flexibility and kinking resistance. These and other advantages of the invention will become more apparent from the following detailed description and drawings.

### 5. BRIEF DESCRIPTION OF THE DRAWINGS

Figure 1 is a plan view of an exemplary embodiment of a catheter in accordance with the present invention.
Figure 2 is a cross-sectional view of the distal portion of the catheter illustrating an alternative tip design.
Figure 3 is a cross-sectional view of the distal portion of the catheter illustrating another alternative tip design.

### 6. DETAILED DESCRIPTION OF PREFERRED EMBODIMENTS

Reference will now be made in detail to the present preferred embodiments of the invention, an example of which is illustrated in the accompanying drawings. The method and corresponding steps of the invention will be described in conjunction with the detailed description of the system.

The devices and methods presented herein may be used for treating the lumenal systems of a patient. The present invention is particularly suited for treatment of the cardiovascular system of a patient, such as performance of angioplasty and delivery of balloon-expandable or self-expanding interventional devices (e.g., stents, filters, coils).

In accordance with the invention, a catheter is provided including an outer tubular member having a length, an outer surface, an inner surface and a lumen therethrough. The catheter also includes an inner tubular member having an outer surface, an inner surface and a lumen therethrough, at least a length of the inner lumen is disposed in the lumen of the outer tubular member. The catheter further includes an inflatable member disposed adjacent the distal end of the outer tubular member and a distal tip.

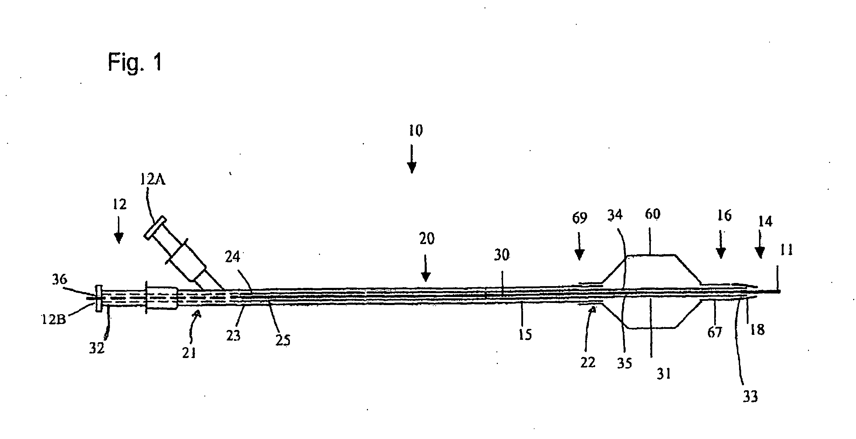

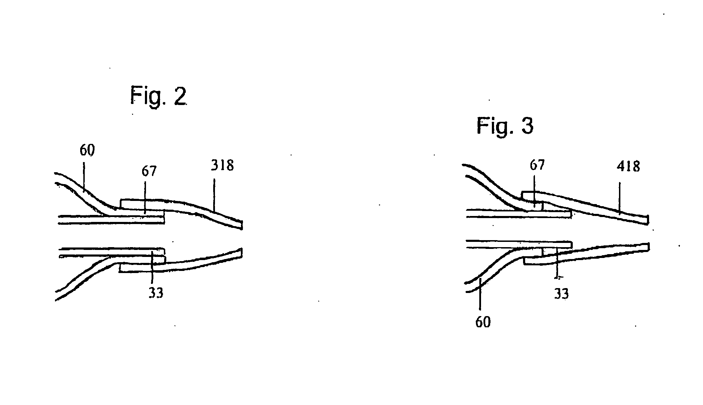

For purpose of explanation and illustration, and not limitation, a side view of an exemplary embodiment of the catheter in accordance with the invention is shown in Fig. 1 and is designated generally by reference character 10. Other embodiments of a catheter in accordance with the invention, or aspects thereof, are provided in Figs. 2-3, as will be described.

For purposes of illustration and not limitation, as embodied herein and as depicted in Fig. 1, catheter 10 is provided with an outer tubular member 20. Outer tubular member 20 has a proximal end 21, a distal end 22, a length L, an outer surface 23, an inner surface 24 and defines a lumen 25 therethrough.

Outer tubular member 20 can be made from a variety of materials, including metal, plastic and composite materials. Metal tubes such as stainless steel hypotubes can be used, and may or may not be coated with a polymeric material such as PTFE. Multilayered polymeric tubes can also be used formed by coextrusion, dipping processes, or by shrinking tubing layers over one another over a mandrel. Moreover, polymeric tubular members can also be formed by charging a mandrel with static electricity, applying plastic in powder or granular form to the mandrel to form a layer of plastic over the mandrel, and by heating the mandrel to cause the particles to fuse. Multilayered polymeric tubes can also be used that include metallic or nonmetallic braiding within or between layers of the tube. A carbon tube can also be used, as well as fiber-reinforced resin materials. If the catheter is only comprised of a single outer tubular along its length, it may be desirable in certain instances to design outer tubular member 20 to have a decreasing stiffness along its length from proximal end 21 to distal end 22.

In further accordance with the invention, a catheter is provided further including an inner tubular member.

For purposes of illustration and not limitation, as embodied herein and as depicted in Fig. 1, catheter 10 includes inner tubular member 30. Inner tubular member 30 has a proximal end 32, a distal end 33, an outer surface 34, an inner surface 35 and defines a lumen 36 therethrough. In accordance with a particular embodiment of the invention depicted in Figure 1, at least a length of the inner tubular member 30 is disposed in the lumen 25 of the outer tubular member 20.

In accordance this aspect of the invention, the inner tubular member 30 can function as a guidewire lumen, as the low friction inner surface 35 of inner tubular member permits a guidewire to move easily through lumen 36. The inner tubular member 30 is disposed within at least a portion of the lumen 25 of the outer tubular member and thereby forming an annular space between the outer surface 34 of the inner tubular member and the inner surface 24 of the outer tubular member 20.

A variety of materials can be used for inner tubular member 30. For example and not limitation, inner tubular member 30 can be made from the same materials as the outer tubular member 20. In accordance with a specific embodiment of the invention, a multilayered tube is used for inner tubular member 30 including a nylon outer layer and an inner layer formed from a lubricious material such as polyethylene of varying densities, PTFE, polyimide, PEEK or PVDF, PEBAX, Nylon, PE, PET, PU and HDPE alone, in blends or in multilayered members. It is further contemplated that the inner tubular member 30 may be constructed of one tube or from two or more composed tube parts, the different parts may consist of different materials.

In a preferred embodiment, the guide wire lumen tube is composed from 2 materials. The proximal portion is formed from a first material, the distal portion of the guide wire lumen tube is formed from a second material more flexible than the first material. Such an arrangement results in a particularly flexible design of the distal end of the guide wire tube and of the catheter. The catheter is highly flexible while still maintaining trackability and pushability. Preferably, the transition portion of the two guide wire lumen tubes is arranged at the proximal portion 69 of the inflatable member 60.

As illustrated in Figure 1, the catheter 10 further includes a hub 12 disposed at the proximal end 21 of the outer tubular member 20, the hub includes a first lumen 12A and a second lumen 12B, wherein the first lumen 12A is in fluid communication with the annular space between the inner surface 24 of the outer tubular member 20 and the outer surface 34 of the inner tubular member 30. The second lumen 12B is in fluid communication with the lumen 36 of the inner tubular member. As shown in Figure 1, a guidewire 11 may be disposed through the second lumen of the hub 12 and through the lumen 36 of the inner tubular member 30, thereby allowing the catheter 20 to be tracked over the guidewire 11 to be placed within a tubular body such as a vessel or artery.

For purposes of illustration and not limitation, as depicted in Fig. 1, the invention, inflation lumen can be used to direct inflation fluid to an inflatable member 60 in fluid communication with the inflation lumen.

Inflatable member 60 can be made from a variety of materials. For purpose of illustration and not limitation, inflatable member 60 can be made from a poly ether block amide ("PEBA"), nylon, Hytrel, PU, PEEK, PE or a variety of other materials. Inflatable member 60 can be attached to distal end 22 of outer tubular member 20 of catheter 10 by way of adhesive bond, fusion bond, or preferably by welding, as described in U.S. Patent Application Serial No. 10/952,543. Thus, if inflatable member 60 is made of nylon, it is advantageous for outer tubular member 20 to be made of a material compatible for a welded bond therebetween.

By way of further example, an inflation device (not shown) is provided for inflating the inflatable member 60. The inflation device (not shown) can be, for example, a syringe or a flexible reservoir that is connected to the first lumen 12A of the hub 12 coupled to the proximal end 21 of outer tubular member 20 and actuated by the physician to inflate the inflatable member 60.

As described above, the inflatable member 60 is disposed on the distal end 22 of the outer tubular member 20, wherein the proximal section 69 of the inflatable member 60 is bonded to the outer surface 23 of the outer tubular member 20. The distal section 67 of the inflatable member 60 is bonded to the outer surface 35 of the inner tubular member 30 in the distal end region of the inner tubular member 30.

As shown in Figure 1, the inflatable member 60 spans the length between the distal end 22 of the outer tubular member 20 and the distal end 14 of the catheter 10.

Further in accordance with the present invention, the catheter 10 includes a distal tip member, wherein the distal tip member may be formed in many different configurations as will be shown in Figures 1-3.

Referring now to Figure 2, there is shown an alternative exemplary embodiment of a distal tip design 318 end of the catheter. As shown in Figure 3, the alternative tip 318 is formed wherein the distal section 69 of the inflatable member 60 is attached to the distal portion 33 of the inner tubular member 30, the distal end 33 of the inner tubular member 30 extending up to the distal section 67 of the inflatable member 60. The tip portion 318 is fixedly attached to the outer surface of the distal portion 67 of the inflatable member 60, thereby forming a softened tip potion.

Referring now to Figure 3, there is shown yet another exemplary embodiment of an alternative tip design. As illustrated in Fig. 3, the distal section 67 of the inflatable member 60 is fixedly attached to the distal portion 33 of the inner tubular member 30, the distal end 33 of the inner tubular member 30 extending distally from the distal section 67 of the inflatable member 60. The tip portion 418 being fixedly attached to the outer surface of the distal portion 69 of the inflatable tubular member 60 and extends distally from the distal portion 67 of the inflatable member 60 and the inner tubular member 30. In this embodiment, the tip portion 418 is bonded to the outer surface of the distal portion 67 of the inflatable member 60 as well as to the outer surface 34 of the distal portion 33 of the inner tubular member 30 extending distally from the distal portion 67 of the inflatable member 60.

In accordance with the present invention and each of the embodiments described in detail above, the tip member of the catheter 10 is preferably formed from a material softer than that of which the inner tubular member is formed in order to provide sufficient flexibility of the catheter tip to allow safe navigation through tortuous anatomy of the patient's vessels but at the same time to provide sufficient pushability and trackability of the catheter to allow crossing of the stenotic vessel sections to be treated.

It is further contemplated though not shown that the tip member may be coated with an abrasive material in order to enable the physician to cross narrow lesions or even total occlusions in the vessels. In another embodiment it is contemplated that the tip material is dyed with a radiopaque material or doped with a radiopaque material to form a tip visible under X-ray.

It would be apparent to the person skilled in the art that the tip member can be made from the same or different material as the balloon and/or the guide wire lumen is made of. Preferably, the tip member in accordance with the present invention is made of PEBAX, Nylon, PE, PET, PU, or blends thereof, or compositions like multilayers, thereof.

In accordance with the present invention herein, it is contemplated that the tip member may be formed in accordance with the methods described herein.

In an alternative embodiment the tip member 18 is positioned at at least a portion of the distal portion 69 of the inflatable member 60, the tip member 18 extending distally from the distal portion 69 of the inflatable member 60 and the inner tubular member 30 to form the tip portion, the tip member, the distal portion 69 of the inflatable member 60 and the inner tubular member 30 are bonded to each other. In this embodiment, the guidewire lumen 36 can extend distally of the inflatable member 60 as shown in Fig. 3, resulting in attachment of the tip member to the outer surface 34 of the distal portion 69 of the inflatable member 60 and the outer surface 34 of the inner tubular member 30. In the alternative design as shown in Fig. 2, the tip member 18 is merely attached to the outer surface 34 of the distal portion 69 of the inflatable member 60, while the distal portion 69 of the inflatable member 60 is attached to the outer surface 34 of the inner tubular member 30.

In order to attach the tip member 18 to the catheter assembly in this alternative embodiment, the inner tubular member 30 is arranged in the desired position relatively to the distal portion 69. A proximal portion of the tip member 18 is pulled over the distal portion of the inner tubular member 30 and at least a portion of the distal portion 69 of the inflatable member 60. The tip member 18, the distal portion 69 of the inflatable member 60 and the inner tubular member 30 are fusion bonded in a one step process by applying heat to at least a portion of the proximal tip portion and the distal portion 69 of the inflatable member 60, to form a balloon catheter tip wherein the tip member is attached to the outer surface of the distal portion 69 of the inflatable member 60.

Typically, for all of the methods of forming the tip portion described above, a mandrel is inserted into the distal guide wire tube and the tip member before fusion bonding is performed. Optionally, a shrink tube can be imposed on the distal balloon shaft and the tip member to achieve good heat distribution and a defined final outer diameter of the tip portion. Both, the mandrel and the shrink tube will be removed after the bonding process is finished. The fusion bonding can be performed by various welding processes including but not limited to contact welding, hot air welding, laser welding, inductive welding, and white light welding technology (shown and described in co-pending US Patent Application serial number 10/952,543. In a preferred embodiment, the fusion bonding is done by transition bonding.

The methods of forming the tip portion of the catheter of the present invention allow the formation of an extremely smooth transition of the balloon cone to the distal tip having a soft taper of the tip portion. Further, all methods allow the formation of the tip portion in a one step process, thus, no successive bonding steps are needed.

One skilled in the art would readily appreciate that the present invention is well adapted to carry out the objects and obtain the ends and advantages mentioned, as well as those inherent therein. The methods, processes, and devices described herein are presently representative of preferred embodiments, are exemplary, and are not intended as limitations on the scope of the invention. It will be readily apparent to one skilled in the art that varying substitutions and modifications may be made to the invention disclosed herein without departing from the scope of the invention.

All patents and publications mentioned in the specification are indicative of the levels of those skilled in the art to which the invention pertains.

The catheter 10 shown in Figure 1 and described herein is an over-the-wire type catheter. However, the catheter of the present invention may be a rapid exchange catheter as well, wherein the proximal guide wire lumen port is located at a position distal of the proximal end of the catheter. In one embodiment of the present invention the guide wire lumen and inflation lumen are arranged coaxially to each other. However, the guide wire lumen and inflation lumen can be arranged side-by-side as well.

The method according to the present invention can provide that the tip portion is formed by heat welding or by laser welding.

Furthermore, the inventive method can provide that a mandrel is inserted into the guidewire lumen of the inner tubular member and the tip member before bonding is performed.

Moreover, in accordance with the method according to the present invention, a shrink tube can be imposed on the distal balloon shaft and the tip member before bonding is performed.

Finally, the method according to the present invention can include the step of providing a luer, the luer attached to the proximal end of the outer tubular member and to the proximal end of the inner tubular member, the luer further including a first lumen and a second lumen, the first lumen being in fluid communication with the balloon, and the second being in communication with the guidewire lumen.

## Claims

1. A balloon catheter (10) comprising:
- an outer tubular member (20) having a proximal end (21), a distal end (22), a proximal shaft section, a distal shaft section;
- an inner tubular member (30) extending within at least a distal portion of the outer tubular member (20), and spaced apart from at least a portion of the outer tubular member (20) to form an inflation lumen therebetween;
- a balloon (60) disposed adjacent the distal end of the catheter (10) and having an inflatable interior in fluid communication with the inflation lumen, a proximal balloon shaft section coupled to the distal portion of the outer tubular member (20), a distal balloon shaft section (67) being bonded to the inner tubular member (30); and
- a tip member (318; 418) extending from a distal end of the inner tubular member (30), the tip member associated with the distal balloon shaft section,
**characterized in that**
- a portion of the tip member (318; 418) is disposed radially about the distal balloon shaft section (67), the distal balloon shaft section (67) being affixed to the distal end (33) of the inner tubular member (30).

2. The balloon catheter according to claim 1, wherein the distal portion (67) of the catheter (10) is more flexible than a proximal portion of the catheter (10).

3. The balloon catheter according to claim 1, wherein the tip member (318; 418) is constructed of a material chosen from the group consisting of PEBAX, Nylon, PE, PET, PU, blends thereof, compositions, and multilayers thereof.

4. The catheter according to claim 1, wherein the inflation lumen and the guide wire lumen are arranged coaxially to each other.

5. The catheter according to claim 1, wherein the inflation lumen and the guide wire lumen are arranged side-by-side to each other.

6. The catheter according to claim 1, wherein the tip portion (418) is bonded to the outer surface (34) of the distal portion (67) of the balloon (60) as well to the outer surface of the distal portion (33) of the inner tubular member (30) extending distally from the distal portion (67) of the balloon (60).

7. A method for manufacturing a balloon catheter (1), the method comprising the steps of:
- providing an elongated outer tubular member (2a) having a proximal end, a distal end, a proximal shaft section, a distal shaft section;
- providing an inner tubular member (3a) extending along and through at least a distal portion of the catheter shaft to a port at the distal end of the outer tubular member and forming an inflation lumen therebetween;
- providing a balloon (60) having a proximal shaft section and a distal shaft section, the proximal shaft section affixed to the distal end of the outer tubular member and having an inflatable interior in fluid communication with the inflation lumen, the distal balloon shaft section affixed to an outer surface of the inner tubular member;
- positioning a tip member (318; 418) at the outer surface inner tubular member at a position juxtaposed to a distal end of the distal balloon shaft section in such a way that the tip member extends distally from the balloon shaft and the guide wire lumen tube; and
- forming the tip portion by bonding the tip member (318; 418), the balloon shaft section and the inner tubular member to each other, and
- disposing a portion of the tip member (318; 418) radially about the distal balloon shaft section (67), the distal balloon shaft section (67) being affixed to the distal end (33) of the inner tubular member (30).

8. Method according to claim 7 comprising the step of:
- bonding the tip portion (418) to the outer surface (34) of the distal portion (67) of the balloon (60) as well as to the outer surface of the distal portion (33) of the inner tubular member (30) extending distally from the distal portion (67) of the balloon (60).

## Patentansprüche

1. Ballonkatheter (10):
- mit einem äußeren Rohrelement (20) mit einem proximalen Ende (21), einem distalen Ende (22), einem proximalen Schaftbereich, einem distalen Schaftbereich;
- mit einem inneren Rohrelement (30), das sich innerhalb zumindest eines distalen Bereichs des äußeren Rohrelements (20) erstreckt, und von zumindest einem Bereich des äußeren Rohrelements (20) beabstandet ist, um ein Inflationslumen dazwischen zu bilden;
- mit einem Ballon (60), der benachbart zum distalen Ende des Katheters (10) angeordnet ist und einen inflatierbaren Innenraum in flüssigkeitsmäßiger Verbindung mit dem Inflationslumen, einen proximalen Ballon-Schaftbereich, der mit dem distalen Bereich des äußeren Rohrelements (20) verbunden ist, einen distalen Ballon-Schaftbereich (67), der mit dem inneren Rohrelement (30) verbunden ist, aufweist; und
- mit einem Spitzenelement (318; 418), das sich von einem distalen Ende des inneren Rohrelements (30) erstreckt, wobei das Spitzenelement mit dem distalen Ballon-Schaftbereich verbunden ist,
**dadurch gekennzeichnet, dass**
- ein Bereich des Spitzenelements (318; 418) radial um den distalen Ballon-Schaftbereich (67) herum angeordnet ist, wobei der distale Ballon-Schaftbereich (67) am distalen Ende (33) des inneren Rohrelements (30) befestigt ist.

2. Ballonkatheter gemäß Anspruch 1, wobei der distale Bereich (67) des Katheters (10) flexibler als ein proximaler Bereich des Katheters (10) ist.

3. Ballonkatheter gemäß Anspruch 1, wobei das Spitzenelement (318; 418) aus einem Werkstoff hergestellt ist, das aus der Gruppe ausgewählt wird, die aus PEBAX, Nylon, PE, PET, PU, Mischungen, und Multilayer davon besteht.

4. Katheter gemäß Anspruch 1, wobei das Inflationslumen und das Führungsdrahtlumen koaxial zueinander angeordnet sind.

5. Katheter gemäß Anspruch 1, wobei das Inflationslumen und das Führungsdrahtlumen nebeneinander angeordnet sind.

6. Katheter gemäß Anspruch 1, wobei der Spitzenbereich (418) mit der Außenfläche (34) des distalen Bereichs (67) des Ballons (60) sowie auch mit der Außenfläche des distalen Bereichs (33) des inneren Rohrelements (30), das sich vom distalen Bereich (67) des Ballons (60) distal erstreckt, verbunden ist.

7. Verfahren zum Herstellen eines Ballonkatheters (1), wobei das Verfahren folgende Schritte aufweist:
- Vorsehen eines verlängerten äußeren Rohrelements (2a) mit einem proximalen Ende, einem distalen Ende, einem proximalen Schaftbereich, einem distalen Schaftbereich;
- Vorsehen eines inneren Rohrelements (3a), das sich entlang und durch mindestens einem distalen Bereich des Katheterschafts zu einer Öffnung am distalen Ende des äußeren Rohrelements erstreckt und ein Inflationslumen dazwischen bildet;
- Vorsehen eines Ballons (60) mit einem proximalen Schaftbereich und einem distalen Schaftbereich, wobei der proximale Schaftbereich, der am distalen Ende des äußeren Rohrelements befestigt ist und einen inflatierbaren Innenraum in flüssigkeitsmäßiger Verbindung mit dem Inflationslumen, und den distalen Ballon-Schaftbereich, der an einer Außenfläche des inneren Rohrelements befestigt ist, aufweist;
- Positionieren eines Spitzenelements (318; 418) an der Außenfläche des inneren Rohrelements an einer Position, die an ein distales Ende des distalen Ballon-Schaftbereichs in der Weise angrenzt, dass sich das Spitzenelement distal vom Ballonschaft und dem Führungsdrahtlumenrohr erstreckt; und
- Bilden des Spitzenbereichs durch Verbinden des Spitzenelements (318; 418), Ballon-Schaftbereichs und inneren Rohrelements miteinander, und
- Anordnen eines Bereichs des Spitzenelements (318; 418) radial um den distalen Ballon-Schaftbereich (67) herum, wobei der distale Ballon-Schaftbereich (67) am distalen Ende (33) des inneren Rohrelements (30) befestigt ist.

8. Verfahren gemäß Anspruch 7, das den folgenden Schritt aufweist:
- Verbinden des Spitzenbereichs (418) mit der Außenfläche (34) des distalen Bereichs (67) des Ballons (60) sowie mit der Außenfläche des distalen Bereichs (33) des inneren Rohrelements (30), das sich vom distalen Bereich (67) des Ballons (60) distal erstreckt.

## Revendications

1. Cathéter à ballonnet (10), comprenant :
- un élément tubulaire extérieur (20) ayant une extrémité proximale (21), une extrémité distale (22), une partie de tige proximale, une partie de tige distale ;
- un élément tubulaire intérieur (30) s'étendant dans au moins une partie distale de l'élément tubulaire extérieur (20), et espacé d'au moins une partie de l'élément tubulaire externe (20) de façon à former un lumen de gonflement entre eux ;
- un ballonnet (60) disposé sur le plan adjacent à l'extrémité distale du cathéter (10) et ayant un intérieur gonflable en communication de fluide avec le lumen de gonflement, une partie de tige de ballonnet proximale couplée à la partie distale de l'élément tubulaire extérieur (20), une partie de tige de ballonnet distale (67) liée à l'élément tubulaire intérieur (30) ; et
- un élément d'embout (318 ; 418) s'étendant d'une extrémité distale de l'élément tubulaire intérieur (30), l'élément d'embout étant associé à la partie de tige du ballonnet distal,
**caractérisé en ce que**
- une partie de l'élément d'embout (318 ; 418) est disposée radialement autour de la partie de tige distale du ballonnet (67), la partie de tige distale du ballonnet (67) étant fixée à l'extrémité distale (33) de l'élément tubulaire interne (30).

2. Cathéter à ballonnet selon la revendication 1, dans lequel la partie distale (67) du cathéter (10) est plus flexible qu'une partie proximale du cathéter (10).

3. Cathéter à ballonnet selon la revendication 1, dans lequel l'élément d'embout (318 ; 418) est constitué d'un matériau choisi dans le groupe comprenant le PEBAX, le nylon, le PET, le PU, des mélanges de ceux-ci, des compositions et des multicouches de ceux-ci.

4. Cathéter selon la revendication 1, dans lequel le lumen de gonflement et le lumen du fil de guidage sont disposés de façon co-axiale l'un par rapport à l'autre.

5. Cathéter selon la revendication 1, dans lequel le lumen de gonflement et le lumen du fil de guidage sont disposés côte à côte l'un par rapport à l'autre.

6. Cathéter selon la revendication 1, dans lequel la partie d'embout (418) est liée à la surface extérieure (34) de la partie distale (67) du ballonnet (60) ainsi qu'à la surface extérieure de la partie distale (33) de l'élément tubulaire interne (30) s'étendant sur le plan distal par rapport à la partie distale (67) du ballonnet (60).

7. Procédé de fabrication d'un cathéter à ballonnet (1), le procédé comprenant les étapes suivantes :
- fournir un élément tubulaire extérieur allongé (2a) ayant une extrémité proximale, une extrémité distale, une partie de tige proximale, une partie de tige distale ;
- fournir un élément tubulaire interne (3a) s'étendant le long et au travers d'au moins une partie distale de la tige de cathéter jusqu'à un orifice au niveau de l'extrémité distale de l'élément tubulaire extérieur et formant un lumen de gonflement entre eux ;
- fournir un ballonnet (60) ayant une partie de tige proximale et une partie de tige distale, la partie de tige proximale étant fixée à l'extrémité distale de l'élément tubulaire extérieur et ayant un intérieur gonflable en communication de fluide avec le lumen de gonflement, la partie de tige distale du ballonnet étant fixée à une surface extérieure de l'élément tubulaire intérieur ;
- placer un élément d'embout (318 ; 418) au niveau de la surface extérieure de l'élément tubulaire dans une position juxtaposée à une extrémité distale de la section de tige distale du ballonnet de telle sorte que l'élément d'embout s'étende sur le plan distal de la tige du ballonnet et du tube à lumen pour le fil de guidage ; et
- former la partie d'embout en liant l'élément d'embout (318 ; 418), la partie de tige du ballonnet et l'élément tubulaire intérieur les uns aux autres, et
- disposer une partie de l'élément d'embout (318 ; 418) sur le plan radial autour de la partie de tige distale du ballonnet (67), la partie de tige distale du ballonnet (67) étant fixée à l'extrémité distale (33) de l'élément tubulaire intérieur (30).

8. Procédé selon la revendication 7, comprenant l'étape suivante :
- lier la partie d'embout (418) de la surface extérieure (34) de la partie distale (67) du ballonnet (60) ainsi que la surface extérieure de la partie distale (33) de l'élément tubulaire interne (30) s'étendant sur le plan distal de la partie distale (67) du ballonnet (60).
